# EUROPEAN PATENT SPECIFICATION

(11) **EP 2 204 143 B1**
(45) Date of publication and mention of the grant of the patent: **02.12.2015**
(21) Application number: 10150022.1
(22) Date of filing: 04.01.2010
(51) Int. Cl.: A61F 2/16

(54) **Intraocular lens**
Intraokularlinse
Lentille intraoculaire

(30) Priority: 06.01.2009 JP 2009001091; 31.08.2009 JP 2009199566
(43) Date of publication of application: 07.07.2010
(73) Proprietor: Nidek Co., Ltd., Gamagori-shi Aichi 443-0038 (JP)
(72) Inventor: Nagasaka, Shinji, Gamagori-shi Aichi 443-0038 (JP); Sunada, Tsutomu, Gamagori-shi Aichi 443-0038 (JP)
(74) Representative: Prüfer & Partner GbR European Patent Attorneys

(56) References cited:
- EP-A1- 0 566 461
- WO-A1-97/20523
- WO-A2-2009/143436
- US-A1- 2003 204 257
- US-A1- 2008 058 830

## Description

### Technical Field

The present invention relates to an intraocular lens to be set in an eye of a patient.

### Background Art

As one of operative treatments for cataract, heretofore, there has generally been used a method in which a lens is removed from a patient's eye and then an intraocular lens (IOL) is injected in place of the lens. Such IOL includes an optic part having a refractive power and loop-shaped support parts each having an open end for supporting the optic part in the eye. The IOL is to be injected into the eye through an incision formed in the eye. The IOL is made of a material so flexible as to be folded and so elastic as to restore to an original shape in order to make the incision as small as possible in the eye (see Patent Literature 1). For injection of such IOL, there is known a technique of injecting the IOL in a bent (folded) state into the eye by use of an IOL injection instrument called an injector (see Patent Literature 2). The IOL injected in the folded state into the eye is unfolded in the eye to restore to the original shape and is placed in a lens capsule and others from which a lens has been removed.

From Patent Literature 3 a foldable intraocular lens is known made of soft resin and comprising an optic part and a pair of support parts integrally formed with the optic part for supporting the optic part in an eye, wherein
the support parts include:
roots formed to extend from a periphery of the optic part and located at positions opposite to each other with respect to a center of the optic part, each root radially extending by a predetermined length in a first direction; and
arms each extending in a curve from the corresponding root in a second direction; and
a constricted portion formed on a side of each arm facing the optic part to inwardly fold the arm by a predetermined length from a distal end thereof,
each support part being foldable at the constricted portion serving as a base point when the arm is subjected to stress during a push-out operation of the intraocular lens by use of an injector.

### Citation List

### Patent Literature

[Patent Literature 1] US 2004042073 A1
[Patent Literature 2] EP 1360946 A1
[Patent Literature 3] WO 97/20523 A1

### Summary of Invention

### Technical Problem

When the IOL is folded and unfolded in the eye by use of the injector, the support parts may become entangled on a push-out unit called a plunger for pushing out the IOL and the support parts may swing or deflect at the time when the IOL is unfolded (released) in the eye. Entanglement of the support parts on the push-out unit causes some problems that damage the support parts and disturb appropriate injection of the IOL. Furthermore, the swing of the support parts caused when the IOL is unfolded is likely to induce unnecessary touching of the support parts with intraocular tissues such as a posterior capsule. It is therefore desired restrain the swing.

The present invention has been made to solve the above problems and has a purpose to provide an IOL capable of being injected appropriately and restraining swing of support parts in an eye during injection of the IOL by use of an injector.

### Solution to Problem

To achieve the above object, the present invention provides a foldable intraocular lens made according to claim 1.

Further developments of the present invention are given in the dependent claims.

### Advantageous Effects of Invention

According to the invention, an IOL can be appropriately ejected out and swing of support parts in an eye can be restrained during injection of the IOL by use of an injector.

### Brief Description of Drawings

FIGs. 1A and 1B are views showing a configuration of an IOL in an example not forming part of the invention;
FIGs. 2A and 2B are views showing an external appearance of an IOL injection instrument in the example not forming part of the invention;
FIG. 3 is a view showing an external appearance of a cartridge in the example not forming part of the invention;
FIGs. 4A and 4B are views showing how to fold the IOL by transform of the shape of the cartridge;
FIG. 5 is a view showing an external configuration of a cylindrical unit;
FIG. 6 is a view showing a side sectional view of the IOL injection instrument;
FIGs. 7A, 7B, and 7C are views for explaining a folding manner of the IOL not forming part of the invention;
FIGs. 8A, 8B, and 8C are explanatory views showing an example not forming part of the invention;
FIG. 9 is an explanatory view showing a configuration of an IOL in an embodiment;
FIG. 10 is an explanatory view showing a configuration of an IOL in a modified example of the embodiment; and
FIGs. 11A and 11B are explanatory views showing a push-out operation of the IOL.

### Description

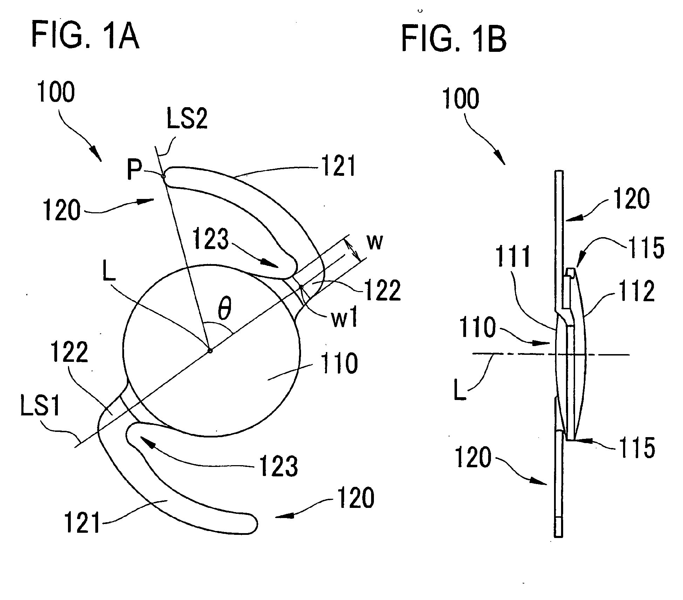

FIGs. 1A and 1B are explanatory views showing a configuration of an intraocular lens (IOL) 100. Specifically, FIG. 1A is a front view and FIG. 1B is a side view. In FIG. 1B, a left side in the drawing sheet is assumed to be a front side of the IOL 100. The IOL 100 includes an optic part 110 having predetermined refractive power and a pair of support parts 120 for supporting the optic part 110 in a patient's eye. Each of the support parts 120 has a loop shape with one open end.

The optic part 110 and the support parts 120 are made of a foldable material commonly used for a typical soft intraocular lens (soft resin), for example, a monomeric material such as hydroxyethyl methacrylate (HEMA) and a composite material of acrylic ester and methacrylate ester. The IOL 100 is formed as a one-piece intraocular lens made of the above intraocular lens material by integrally forming the optic part 110 and the support parts 120 by cutting, molding, or the like.

The optic part 110 is a disc-shaped member including a front surface 111 formed as a convex surface having a predetermined curvature and a back surface 112 formed as a convex surface having a predetermined curvature and formed with an edge portion 115 in an outer circumferential portion (a peripheral portion). The edge portion 115 is formed at least on the back side of the periphery of the optic part 110 and has such a shape (an angle) as to bite in a posterior capsule of the eye when the IOL 100 is placed in the capsule. Close contact of the edge portion 115 with the capsule will restrain secondary cataract. To be concrete, the edge portion 115 making contact with the capsule prevents cells wandering or migrating from the vicinity of the optic part 110 from going around to the back surface 112 side. This prevents clouding of cells of the posterior capsule behind the back surface 112.

Each support part 120 is formed from a part of the periphery of the optic part 110. The two support parts 120 are formed in a pair to be opposite each other relative to an optical axis L. Each support part 120 includes an arm 121 for supporting the optic part 110 in the capsule and a root 122 connecting the optic part 110 and the arm 121. One end of the arm 121 is free and the other end is continuous with the root 122. A pair of the roots 122 is placed oppositely with respect to the center (the optical axis L) of the optic part 110. Each root 122 is formed to extend radially (in a first direction) by a predetermined length from the periphery of the optic part. Each arm 121 is bent from the root 122 at a predetermined angle around the optical axis L, and extends in an inward curve in a second direction toward a distal end. Each arm 121 has a curvature radius slightly larger than a curvature radius of an outer circumference of the optic part 110. The "second direction" indicates a direction in which each arm 121 almost entirely comes into contact with the edge (equator) of the capsule of the patient's eye.

The length of each arm 121 (the length of each arm 121 from a joining portion with the root 122 to the distal end) is determined to be such a length as that the distal end of the folded support part 120 is located inside the folded optic part 110. To be concrete, the length of each arm 121 is determined to provide an angle θ of 50° or more and 90° or less between a line segment LS1 joining a midpoint w1 of the width w of the root 122 (the width defined by a line segment almost perpendicular to the first direction in which the root 122 extends) to the center (the optical axis L) of the optic part 110 and a line segment LS2 joining a distal end P of the arm 121 to the center of the optic part 110. If the angle θ is less than 50°, the contact between the lens capsule and the support part 120 is insufficient for stably supporting the IOL 100 in the eye. If the angle θ exceeds 90°, the arm 121 is too long to likely cause entanglement with the plunger and others during use of an injector mentioned later.

An outermost diameter (a maximum total length) of the IOL 100 is determined to be so long as to be subjected to an appropriate stress from outside to inside of the support part 120 when the IOL 100 is placed in the capsule. The outermost diameter is preferably 9 mm or more and 15 mm or less. The diameter of the optic part 110 is set based on the size of a pupil, preferably, 4 mm or more and 8 mm or less. The shape of the support part 120 is determined to meet those conditions in addition to the aforementioned angle θ condition. The length of each root 122 is determined so that the length between the distal ends of the oppositely disposed roots 122 is equal to or less than the diameter of the capsule of the patient's eye, preferably, almost equal to the diameter (8 mm to 10 mm) of the capsule of the patient's eye. Accordingly, when the IOL 100 is placed in the capsule, the optic part 110 is unlikely subjected to stress from the capsule. For instance, the optic part 110 remains unlikely to move backward and forward even after a long time use.

The IOL 100 is configured to be folded into a smaller size by use of the injector mentioned later so that the support parts 120 are compressed toward the optic part 110 when a distance between inner walls of the injector is narrowed and the optic part 110 is folded with the support parts 120 remaining held on the optic part 110. Therefore, each support part 120 is designed so that the arm 121 has such a length as to fall within the diameter of the optic part 110 when the arm 121 (the support part 120) is compressed to fold the IOL 100.

Furthermore, an angle between the first direction and the second direction (a tangent line at an inflection point) to bend each arm 121 from the root 122 is determined to an obtuse angle of 90° or more and here almost 90°. At a boundary of the inside (the side facing the optic part) of the arm 121 and the root 122, a constricted portion 123 is formed. The inside of a proximal end of the arm 121 continuous with the constricted portion 123 is recessed toward the outer circumference. Accordingly, the constricted portion 123 has a shape recessed (constricted) outward from a ridge line of an inside curve of the arm 121. The width of the support part located at the constricted portion 123 is determined to be equal to or shorter than the width (the lateral width) of a midpoint (a middle portion) of the arm 121. In other words, the constricted portion 123 is provided around an intersection of the first and second directions so that stress when applied on the support part 120 (the arm 121) is transmitted to the vicinity of the constricted portion 123, thereby causing the support part 120 to fold at the constricted portion 123 acting as a folding base point (the stress concentrates on the base point). In each support part 120 having the above configuration, the arm 121 can entirely bend from a folded portion (the constricted portion 123) serving as the base point under the stress applied on the distal end. This stress represents a force applied on each arm 121 when the IOL 100 is placed in the capsule.

The support parts 120 are formed of the aforementioned soft resin with the following size to make the arms 122 foldable. Herein, the width is defined as the length of a member in a cross section almost perpendicular to a direction of extension (e.g., the first direction) of the member. The width of each root 122 is preferably 0.5 mm to 2.0 mm. The width of each arm 121 is preferably 0.1 mm to 1.0 mm. It is to be noted that the width of each root 122 is preferably wider by the order of 0.1 mm to 1.0 mm than the width of each arm 121. The width of each arm 121 is determined to 0.4 mm and the width of each root 122 is determined to 0.7 mm. The thickness of each root 122 is preferably 0.1 mm to 1.0 mm and the thickness of each arm 121 is preferably 0.1 mm to 1.0 mm. Desirably, the thickness of each root 122 is equal to or larger than the thickness of each arm 121. The thickness of each root 122 and each arm 121 is determined to 0.45 mm. Alternatively, the thickness of each root 122 may be set to 0.45 mm and the thickness of each arm 121 may be set to 0.35 mm. Another alternative is to form each arm 121 to have the thickness gradually decreasing from the proximal end to the distal end.

In order to make each constricted portion 123 act as the folding base point when each support part 120 is subjected to the stress, each support part 120 is so designed that a transition area (the joining portion) from the root 122 to the arm 121 has an outside curve (outer circumference) at a curvature radius larger than a curvature radius of the inside curve (inner circumference) at the constricted portion 123. Specifically, the curvature radius of the inside curve (the constricted portion 123) is preferably 0.1 mm to 1.0 mm and the curvature radius of the outside curve is preferably 0.5 mm to 2.0 mm. The inside curvature radius is set to 0.38 mm and the outside radius curvature is set to 1.0 mm.

Each support part 120 (each arm 121) is formed to be located on a more front side than the periphery of the optic part 110 in order to press the optic part 110 against the posterior capsule. Since the support parts 120 are positioned on the more front side than the periphery of the optic part 110, the optic part 110 is moved backward when stress is applied from outer circumference to the IOL 100 placed in the capsule. Accordingly, the optic part 110 is appropriately pressed against the posterior capsule, thereby bringing the edge portion 115 into close contact with the posterior capsule.

Since the support parts 120 are located on the more front side than the optic part 110, the support parts 120 are easy to be put on the optic part 110 in a folding operation of the IOL 100 mentioned later.

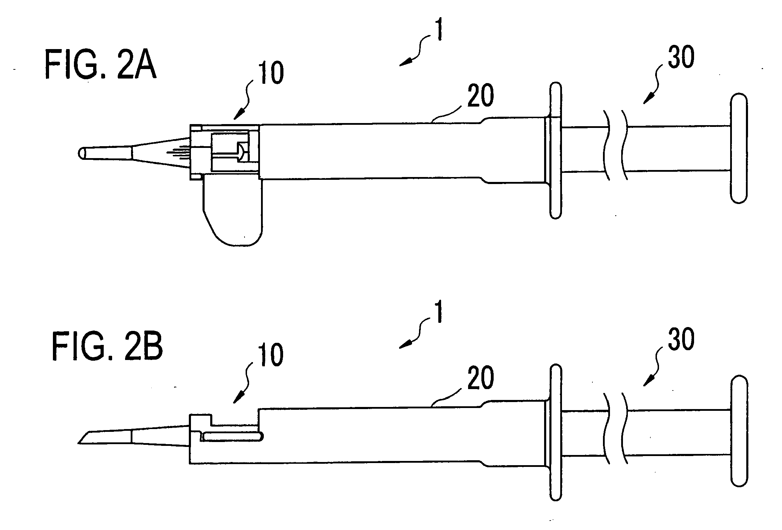

An IOL injection instrument 1 (hereinafter, an "injector") for injecting the aforementioned IOL 100 into the patient's eye will be explained below. FIGs. 2A and 2B are schematic views showing an external appearance of the injector 1 used. Specifically, FIG. 2A is a top view of the injector 1 and FIG. 2B is a side view of the same.

The injector 1 includes, in the order of insertion into the eye, a lens holding part 10 (hereinafter, a "cartridge") serving as a lens cartridge provided with an injection part to be injected in an incision formed in the eye and a setting part on which the IOL 100 is to be set, a cylindrical unit (an injector main body) 20 for mounting the cartridge 10 at a tip end, and a push-out unit (hereinafter, a "plunger") 30 to be inserted through the inside of the cartridge 10 and the cylindrical unit 20 to push out the IOL 100 from the tip end of the cartridge 10 mounted in the cylindrical unit 20.

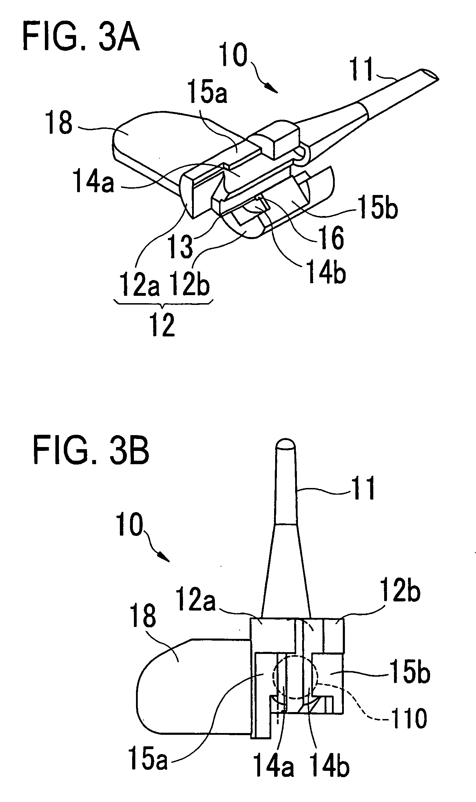

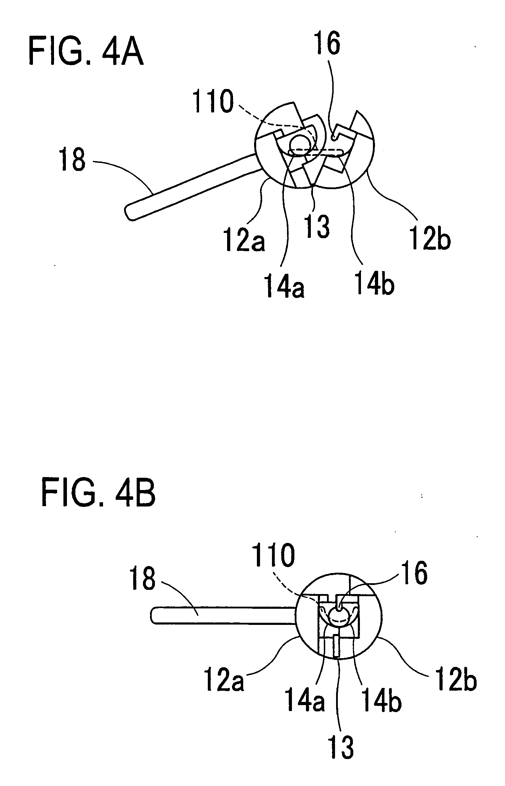

FIGs. 3A and 3B and FIGs. 4A and 4B are views showing a configuration of the cartridge 10. As shown in the figures, the cartridge 10 includes an injection part 11 having a tapered shape whose diameter is gradually smaller (narrower) toward a tip end and a setting part 12 for setting the IOL 100, the injection part 11 and the setting part 12 being integrally joined.

The injection part 11 is formed in a hollow tubular shape through which the folded IOL 100 can be pushed out through a hollow portion thereof. The injector 11 has such an internal shape as to further fold the IOL 100 into a smaller size than that in the setting part 12.

The setting part 12 is constituted of two half-split elements 12a and 12b. As shown in Fig. 3A, the half-split elements 12a and 12b are connected to each other at respective lower edges by a hinge 13 so as to be opened and closed. The half-split elements 12a and 12b include setting stages 14a and 14b respectively for mounting thereon the IOL 100. The shapes (wall shapes) of the setting stages 14a and 14b are curved in a direction to fold the IOL 100.

When the half-split elements 12a and 12b are closed into contact with each other, the shapes of walls (setting surfaces) of the setting stages 14a and 14b are transformed to substantially conform in cross section to the shape (semi-circle) of an opening of the injection part 11 on a rear end side (see Figs. 4A and 4B). The outer shape of the setting part 12 with the half-split elements 12a and 12b being closed is substantially equal to the shape of an inner wall of the cylindrical unit 20 mentioned later.

Covers 15a and 15b are provided as upper portions of the half-split elements 12a and 12b respectively to cover over the setting stages 14a and 14b when the half-split elements 12a and 12b are closed. The cover 15b is provided at an end with a protrusion 16 protruding from above and toward the setting stages 14a and 14b (the hinge 13) and extending by a predetermined length in an axial direction. The protrusion 16 serves to guide a bending or folding direction of the IOL 100 set in the cartridge 10 to a direction along the inner walls (the setting surfaces) of the setting stages 14a and 14b. A flat-plate-like grip 18 is provided on the side of the half-split element 12a. The grip 18 will be grasped by a user to hold the cartridge 10.

As shown in Fig. 4A, the IOL 100 is set on the setting stages 14a and 14b of the cartridge 10 while the setting part 12 is opened (the half-split elements 12a and 12b are separated) so that the pair of support parts 120 of the IOL 100 face the walls of the setting stages 14a and 14b respectively. As the grip 18 (a proximal end side) of the cartridge 10 is engaged in the recess 23 of the mounting part 21, the bottom of the setting part 12 (the half-split elements 12a and 12b) is pressed against the protrusions 22 (or right and left edges of the mounting part 21). When the bottom (the lower part) of the setting part 12 is pressed against the protrusions 22 (or the right and left edges of the mounting part 21), the protrusions 22 will guide the half-split elements 12a and 12b to close into contact with each other. As the setting part 12 is further pushed into the mounting part 21, the half-split elements 12a and 12b are mounted in a closed state in the mounting part 21. By the above operation of mounting the cartridge 10 into the cylindrical unit 20 or a operator's operation of closing the cartridge 10, the half-split elements 12a and 12b are closed as shown in FIG. 4B, the set IOL 100 is folded to a certain degree.

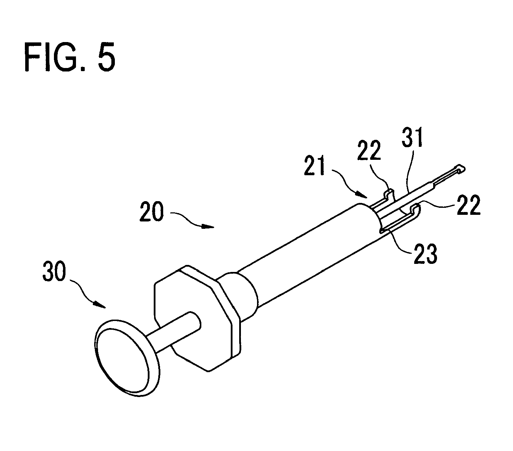

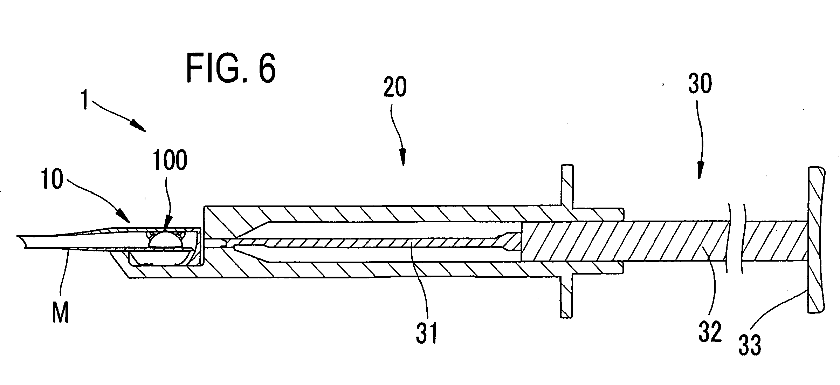

FIG. 5 is a schematic perspective view showing an external appearance of the cylindrical unit 20. The details of an internal structure of this cylindrical unit 20 are explained referring to Fig. 6. As shown in the figures, the cylindrical unit 20 is provided, at a front end, with a mounting part 21 in which the cartridge 10 is to be removably mounted. The mounting part 21 is of a nearly halved shape of the front end portion of the cylindrical unit 20. The mounting part 21 is formed with the recesses 23 in which the grip 18 (the proximal end side) of the cartridge 10 will be engaged and also the protrusions 22 at an opposite side (a cylindrical unit front end side) to the recesses 23. Such mounting part 21 serves as a guide for restricting the opened half-split elements 12a and 12b to close each other when the cartridge 10 is mounted in the mounting part 21 and limiting the width of the setting part 12. The mounting part 21 serves to lock the cartridge 10 in the cylindrical unit 20.

The internal configuration of the injector 1 constituted of a combination of the cartridge 10 and the cylindrical unit 20 will be explained below. FIG. 6 is a schematic sectional view of the injector 1. In FIG. 6, the IOL 100 is in a folded state in the cartridge 10 mounted in the mounting part 21.

The inside of the cylindrical unit 20 is hollow, in which the plunger 30 is inserted so as to be movable forward and backward in an axial direction of the cylindrical unit 20. The plunger 30 includes a push rod 31 whose front end will come into contact with the optic part 110 to push out the IOL 100 through the cartridge 10, a base part 32 provided at one end with the push rod 31 and movable forward and backward in the cylindrical unit 20, and a press part 33 to be pushed from behind by a user. Herein, the support parts 120 are not illustrated for the sake of convenience.

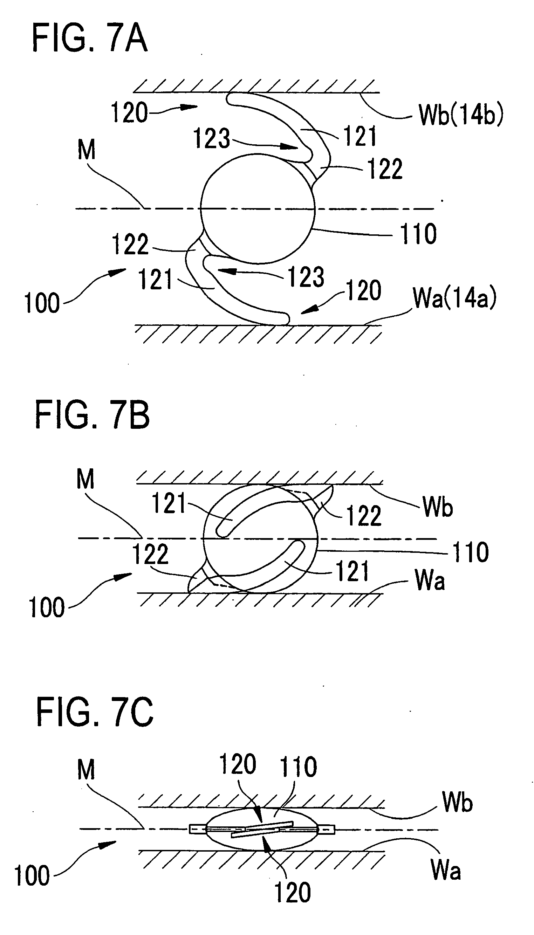

Folding of the IOL 100 will be explained below with reference to FIGs. 7A, 7B, and 7C. The following explains a folding manner of the IOL 100 on the setting stages 14a and 14b when the cartridge 10 is set in the cylindrical unit 20. It is to be noted that signs "Wa" and "Wb" in the figures represent the walls of the setting stages 14a and 14b respectively.

The IOL 100 is placed so that the support parts 120 are located on right and left sides with respect to a push-out axis M (the support parts 120 face the walls of the setting stages 14a and 14b respectively) as shown in FIG. 7A. In this state, the IOL 100 is placed so that an extending direction (a protruding direction) of each root 122 has an angle of about 45° with respect to the push-out axis M. The IOL 100 is further disposed so that the arms 121 are pressed earlier than the roots 122 by the walls Wa and Wb respectively.

When the walls Wa and Wb are narrowed or moved closer to each other with respect to the push-out axis M by the mounting operation of the cartridge 10 into the cylindrical unit 20, the outer sides of the arms 121 are pressed toward the optic part 110 by the walls Wa and Wb. When the distance between the walls Wa and Wb is further shortened, each arm 121 is folded at the constricted portion 123 serving as the base point. The roots 122 are positioned oppositely with respect to the center of the optic part 110 and formed to radially extend by a predetermined length. Accordingly, the IOL 100 is prevented from rotating improperly even when stress is applied to the arms 121 by the walls Wa and Wb. Thus, the set position of the IOL 100 can be maintained appropriately.

As shown in FIG. 7B, in which the walls Wa and Wb are moved to close positions that contact with the optic part 110, the arms 121 are folded toward the optic part 110 by the walls Wa and Wb to overlap the optic part 110. When the distance between the walls Wa and Wb is further narrowed, the optic part 110 begins to fold. At that time, the roots 122 are subjected to stress from the walls Wa and Wb but less deformed as compared with the arms 121. Accordingly, while the arms 121 are held on the optic part 110 as shown in FIG. 7B, the optic part 110 is folded.

In a state where the distance between the walls Wa and Wb are narrowed until the walls Wa and Wb reach respective predetermined positions, that is, in a state where the setting stages 14a and 14b are closed, the optic part 110 is folded with the support parts 120 (the arms 121) being caught in the optic part 110 as shown in FIG. 7C. This prevents each support part 120 from becoming entangled on the push rod 31 or stretched in a passage. The IOL 100 can therefore be pushed out and unfolded appropriately.

The IOL 100 is a one-piece lens but not limited thereto. The IOL has only to have such a shape as to restrain swing of support parts and thus may be a three-piece intraocular lens formed of support parts and an optic part separately produced and assembled into one.

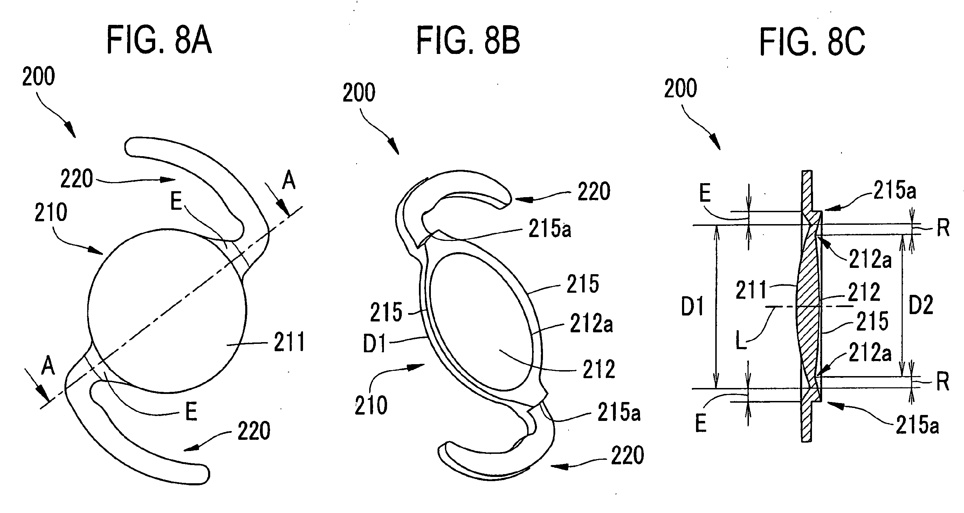

The support parts can be folded together with the optic part when the IOL is folded. However, such IOL tends to be large in size after folding by just the folded support parts.. FIGs. 8A, 8B, and 8C are explanatory views showing a configuration of an IOL 200 with a thinner optic part to enable more appropriate handling. Specifically, FIG. 8A is a front view, FIG. 8B is a perspective back view, and FIG. 8C is a sectional view taken along a line A-A in FIG. 8A.

The IOL 200 has an optic part 210 and support parts 220 as with the aforementioned IOL 100. Herein, the support parts 220 are identical in configuration.

The optic part 210 has a front surface 211 and a back surface 212 each having a curvature to obtain predetermined refractive power. The back surface 212 is formed, at its outer circumference (peripheral edge), with an edge portion 215 having a predetermined height in the optical axis direction. The optic part 210 is configured such that the front surface 211 and the back surface 212 are as near as possible to each other in order to reduce the thickness (in the optical axis direction). To be concrete, as shown in FIG. 8C, a recessed portion 212a is formed between a curved surface of the back surface 212 and the edge portion 215. The back surface 212 is curved from the recessed portion 212a toward the optical axis L. It is to be noted that a slant surface is formed from the edge portions 215 and 215a to the recessed portion 212a.

Herein, to ensure the strength of each support part 220, that is, the strength of a root with respect to the optic part 210, the front surface 211 and the back surface 212 are designed to have different diameters. Specifically, the diameter D2 of the back surface 212 is smaller than the diameter D1 of the front surface 211. Accordingly, the optic part 210 includes an annular portion R resulting from a difference between the diameters D1 and D2. Protruding portions E are formed outside the annular portion R. From these protruding portions E, the support parts 220 are formed. The protruding portions E are provided only at the places of the support parts 220. Thus, each protruding portion E can have a thickness enough to form the support part 220 while the annular portion R retains sufficient thickness, so that the strength of the support part 220 is therefore ensured (see FIG. 8C).

As explained above, the presence of the recessed portion 212a keeps the edge portions 215 and 215a independent of the curved surface 212. The edge portions 215 and 215a can therefore be formed without increasing the thickness of the optic part 210.

As above, the thickness of the optic part 210 is reduced by forming the front surface 211 and the back surface 212 at a short distance while ensuring the strength of the support parts 220. Since the outer diameter of the front surface 211 is larger than the outer diameter of the back surface 212, a recessed portion on the front surface of the optic part 210 is provided only at each root of the support parts 220. When the IOL 200 is to be placed in a capsule or after placed in the capsule, therefore, the optic part 210 is unlikely to disturb the flow of a fluid such as perfusion liquid and aqueous fluid. The shape of each edge portion 215, 215a can be determined regardless of the shape of the back surface 212. Accordingly, even when the optic part 210 is made thinner, a backward protruding edge portion can be formed. For instance, an edge portion can be formed to protrude more backward than the back surface 212. In this case, irrespective of the shape of the optic part 210, the edge portion 215 can be made to efficiently bite into the capsule, thereby preventing secondary cataract.

The shape of each support part is so designed as to be put on the optic part when the IOL is folded but not limited thereto. Any one-piece IOL may be adopted if only it has a thinner optic part. The support parts may have any shape. The IOL is a soft intraocular lens but may be made of a harder material such as PMMA (polymethacrylate) than the aforementioned soft material.

The recessed portion is provided on the back surface of the optic part to reduce the thickness of the optic part. As an alternative, a recessed portion may be provided in a circumferential portion of the front surface of the optic part to make a curved surface of the front surface closer to the back surface, thereby reducing the thickness of the optic part. In this case, the recessed portion formed in the front surface has such a size as not to disturb the flow of a fluid such as perfusion liquid and aqueous fluid.

The IOL is configured such that the support parts are caught in the optic part. However, the IOL may have any other configuration if only it is able to be appropriately injected out by use of an injector and prevent swing of the support parts in the eye. A modified example and operations thereof will be explained below.

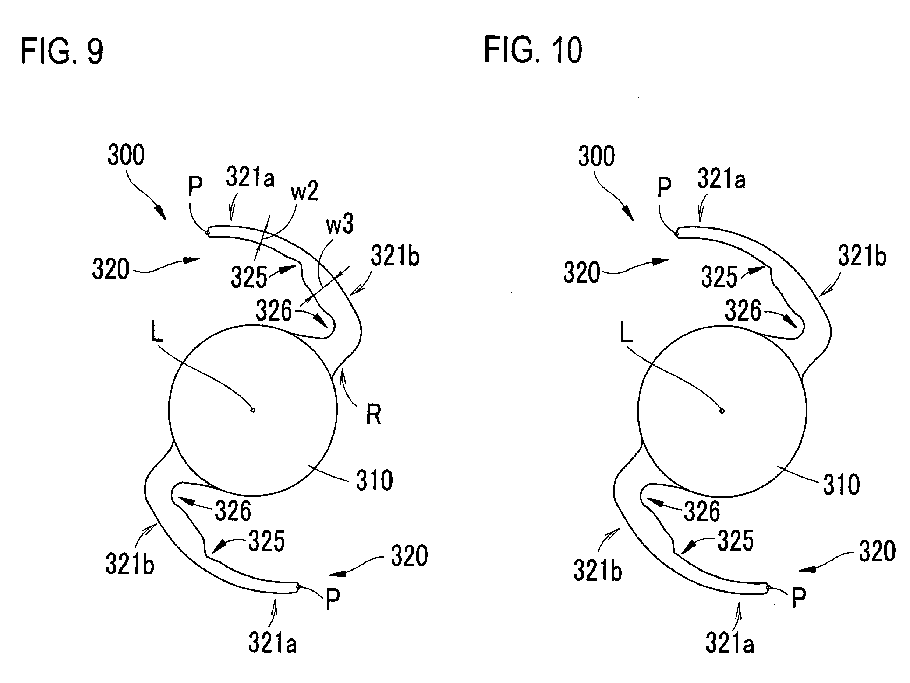

An IOL 300 in FIG. 9 includes an optic part 310 and support parts 320. On the inside of each support part 320 (on the side that will not contact with the capsule when the IOL 300 is set in an eye), a constricted portion 325 is formed between a distal end P and a root R and a constricted portion 326 is formed near the root R joining the support part 320 to the optic part 310. Specifically, the constricted portion 326 is formed between the root R and a rear end portion 321b. The constricted portion 325 is formed between the constricted portion 326 and the distal end P (a distal end portion 321a). The constricted portion 325 is formed on the inside of the distal end portion 321a to allow the distal end portion 321a (arm) to fold inward and put only a part (with a predetermined length from the distal end P) of the distal end portion 321a onto the optic part 310. This predetermined length is determined so as to fall short of the vicinity of the center of the optic part 310. The two constricted portions 325 and 326 are formed by constricting the inside (the inner periphery) of each support part 320 to have a narrower width than other portions of the support part 320 located before and behind each constricted portion.

The constricted portion 325 has an inwardly constricted shape to have a narrower width than the other portions of the support part 320 located before and behind the constricted portion 325 but not limited thereto. The constricted portion 325 has only to be shaped so as to enable the support part 320 to be folded (bent) at a midpoint thereof serving as a base point when a predetermined stress is applied to near the distal end of the support part 320. As shown in FIG. 10, for example, the constricted portion 325 may be provided as an inner peripheral shape changing portion having a shape that the width of the support part 320 extending from the distal end toward the proximal end is maintained by a predetermined length without decreasing halfway and drastically widened therefrom as shown in FIG. 10. This inner peripheral shape changing portion is also included in the constricted portion in this embodiment.

In each support part 320, the width thereof at the constricted portion 325 is determined to be narrower (thinner) than the width at the constricted portion 326. Alternatively, the support part 320 is designed such that, when the support part 320 is sectioned and viewed at each of the constricted portions 325 and 326, the sectional area at the constricted portion 325 is smaller than the sectional area at the constricted portion 326. The sectional area of the constricted portion 326 is a sectional area defined by a bisector at the intersection of the first and second directions.

Assuming that a region from the constricted portion 325 to the distal end P is the distal end portion 321a of the support part 320 and a region from the constricted portion 325 to the constricted portion 326 is the rear end portion 321b of the support part 320, the width of the constricted portion 325 is slightly narrower than the width w2 of the distal end portion 321a and the width of the constricted portion 326 is narrower than the width w3 of the rear end portion 321b. The width w2 of the distal end portion 321a is preferably 0.1 mm to 0.6 mm and more preferably 0.2 mm to 0.5 mm. The width w3 of the rear end portion 321b is determined to be wider than the width w2 and preferably 0.6 mm to 1.5 mm and more preferably 0.7 mm to 1.1 mm. The thicknesses of the distal end portion 321a and the rear end portion 321b (the thickness of the support part 320 in the optical axis direction of the optic part 310) may be equal to each other, but alternatively may be determined so that the thickness of the distal end portion 321a is slightly thinner than that of the rear end portion 321b. The thickness of the distal end portion 321a is preferably 0.2 mm to 0.5 mm and more preferably 0.3 mm to 0.4 mm. The thickness of the rear end portion 321b is preferably 0.3 mm to 0.6 mm and more preferably 0.4 mm to 0.5 mm.

With the above configuration, when a predetermined stress is applied to the distal end of the support part 320, the distal end portion 321a of the support part 320 is folded at the constricted portion 325 serving as the base point. When a predetermined stress is applied uniformly over the distal end portion 321a up to the vicinity of the rear end portion 321b of the support part 320, the support part 320 is entirely folded at the constricted portion 326 serving as a based point.

Supporting that the capsule diameter of the patient's eye is set in advance at a predetermined value (e.g., 10 mm in diameter) as in the aforementioned embodiment, the outermost diameter of the IOL 300 is determined to be sufficiently longer (e.g., about 11 mm to 15 mm) than the predetermined value. At that time, each support part 320 is designed so that an outer periphery from the distal end to a midpoint between the constricted portions 325 and 326 is located more outside than the circumference of a circle (the set capsule diameter) defined by a predetermined value from the center of the optic part 310. It is not necessary to locate the entire outer periphery of each support part 320 formed between the constricted portions 325 and 326 more outside than the set capsule diameter. It may be designed so that about a distal half of the outer periphery of each support part 320 between the constricted portions 325 and 326 is located more outside than the set capsule diameter.

The push-out operation of the IOL 300 in the aforementioned injector 1 will be explained below. A user (an operator) sufficiently fills a viscoelastic material such as hyaluronic acid in the injection part 11 and the setting stages 14a and 14b. This viscoelastic material serves to smoothly move the IOL 300 from the inside of the injector 1 into the capsule.

The user then sets the IOL 300 on the setting stages 14a and 14b. At that time, the IOL 300 is positioned with the front support part 320 being placed on the injection part 11 side. The user holds the cartridge 10 by grasping the grip 18 by one hand, engages the grip 18 (a proximal end side) of the cartridge 10 into the recesses 23 of the mounting part 21 and simultaneously presses the bottom of the setting part 12 (the half-split elements 12a and 12b) against the protrusions 22 (or the right and left edges of the mounting part 21). When the bottom (the lower part) of the setting part 12 is pressed against the protrusions 22 (or the right and left edges of the mounting part 21), the protrusions 22 guide the half-split element 12a and the half-split element 12b to be closed together. As the setting part 12 is further pushed into the mounted part 21, the cartridge 10 is mounted in the mounting part 21 while the half-split elements 12a and 12b are closed together.

While the half-split elements 12a and 12b are closed together, the width (distance) between the setting stages 14a and 14b is narrow and hence the wall surfaces of the setting stages 14a and 14b press against the IOL 300 from right and left. As a result, the IOL 300 is subjected to stress and thus slightly folded along the wall surfaces (the setting surfaces) of the setting stages 14a and 14b.

After the cartridge 10 is mounted in the mounting part 21, the injector 1 is operated to perform an injecting operation of the IOL 300. The push rod 31 of the plunger 30 is moved toward the distal end within the cylindrical unit 20. At that time, when a distal end of the rear support part 320 is pushed by the push rod 31, the rear support part 320 is folded at the constricted portion 325 serving as a base point.

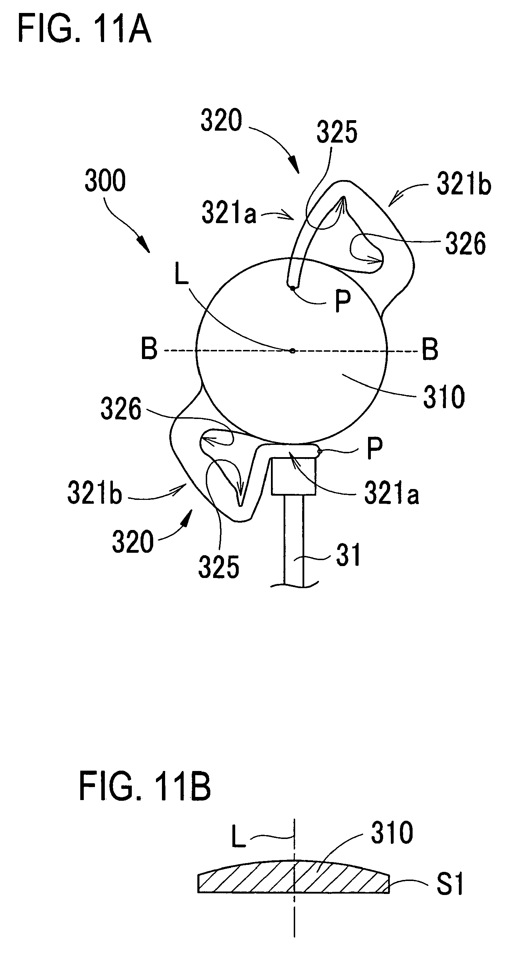

Meanwhile, when the optic part 310 is folded slightly along the wall surfaces (the setting surfaces) of the setting stages 14a and 14b, the support parts 320 may also folded along the wall surfaces of the setting stages 14a and 14b. In this state, as the rear support part 320 is pushed by the push rod 31, part of the distal end portion 321a is caught between the push rod 31 and the edge portion 315 of the optic part 310 (see FIG. 11A). On the other hand, in the case where the support parts 320 are not folded along the wall surfaces, the rear support part 320 is disengaged from the push rod 310 when the push rod 31 makes contact with the edge portion 315 of the optic part 310. Thus, a part of the distal end portion 321a is put on the optic part 310.

Accordingly, the entire IOL 300 is pushed forward to the injection part 11. At this time, the IOL 300 is subjected to pressure in a push-out direction by the viscoelastic material, thereby folding the front support part 320 at the constricted portion 325 serving as a base point. The distal end portion 321a is gradually moved on the peripheral portion of the optic part 310. As above, each support part 320 is folded halfway by the stress generated during the push-out operation.

FIG. 11A shows a state where the distal end P of the support part 320 is put on the optic part 310. In FIG. 11A, the optic part 310 is schematically illustrated as a circle. FIG. 11B is a sectional view taken along a line B-B passing the optical axis L and orthogonal to the push-out direction of the IOL 300. When the IOL 300 is viewed in an orthogonal cross section to the push-out direction, a sectional area S1 at the position of the optical axis L (the center position) of the optic part 310 is a maximum value. Accordingly, when the support part 320 is put on the optic part 310 near the optical axis L, the maximum value of the sectional area of the IOL 300 during a push-out operation is increased by just the thickness of the support part 320. The IOL 300 is thus hard to be injected.

In the present embodiment, therefore, the constricted portion 325 is provided at a point in each support part 320 to cause the distal end portion 321a having a predetermined length from the distal end of the front support part 320 to be first folded in the push-out process. This prevents the distal end P of the support part 320 from folding up to the vicinity of the center of the optic part 310. In the rear support part 320, similarly, the distal end portion 321a is first folded at the constricted portion 325. Thus, when the support part 320 is to be put on the optic part 310, only a part of the distal end portion 321a is just put on the peripheral edge of the optic part 310. The distal end P is avoided from reaching the center of the optic part 310.

As above, even when the IOL 300 is integrally folded with the support part 320 being put on the optic part 310, the maximum value of the sectional area of the IOL 300 during injection is unlikely to increase. In other words, when the distal end portion 321a is folded to a maximum extent, the constricted portion 325 acts to bring the distal end P of the support part 320 to a place other than the center of the optic part 310 and its vicinity.

The IOL 300 is moved into the injection part 11 and further folded (rolled) along the inner wall surface of the injection part 11. The IOL 300 folded in a small size is pushed out through the distal end of the injection part 11 into the eye. At that time, a part of each support part 320 remains on the optic part 310. However, the increasing amount of the maximum value of the sectional area of the IOL 300 is limited and it is unnecessary to increase the size of the incision to be formed in the patient's eye in which the IOL 300 is to be set.

When a force in the forward and backward (push-out) direction is further applied to the IOL 300 with the distal end portion 321a being folded to a maximum extent, the constricted portion 326 is bent by the force exerted thereon and thus the entire support part 320 begins to fold. However, most part of the pressure applied in the forward and backward (push-out) direction has already been exerted on the constricted portion 325. Accordingly, the folding amount of the entire support part 320 at the constricted portion 326 is just small.

When the IOL 300 begins to come out of the distal end of the injection part 11, the optic part 310 and the support parts 320 gradually open. When the IOL 300 is placed in an open state in the capsule, the outside (outer periphery) of each support part 320 substantially fits in the capsule, holding the IOL 300 by a repulsive force generated in the constricted portions 326 through the support parts 320. Each support part 320 is sufficiently long to naturally bend along and contact with the inside of the capsule, under compression concentrically applied from its surrounding. Consequently, the center point of the optic part 310 is not liable to be displaced and thus the entire IOL 300 can be retained appropriately.

The extending degree of the support parts 320 at the constricted portions 326 is determined so that the distance between the distal ends of the oppositely disposed roots R is equal to or smaller than the diameter of the capsule of the patient's eye.

Since the constricted portion 325 is provided in each support part 320, as above, the maximum sectional area of the IOL 300 during injection is not liable to increase even when each support part 320 has a length required for realizing good installation in the capsule. This makes it possible to appropriately fix the IOL 300 in the capsule by the support parts 320 and also reduce the size of an incision to be formed in the patient's eye.

The above example exemplifies the case where the constricted portions 325 and 326 are formed at two places in each support part 320. As an alternative is to form the constricted portions at three or more places in each support part 320. For instance, in the case where each distal end portion 321a is formed with three constricted portions, even though the IOL 300 injected in the capsule is subjected to a concentrically applied force, the constricted portion 325 and the third constricted portion are bent under the concentrically applied force, generating a repulsive force, thus causing each support part 320 to more appropriately contact with the inside of the capsule.

In the IOL 300 explained above, the extending direction of the proximal end portion of each support part 320 extending from the periphery of the optic part 310 is defined as a radial direction from the optical axis of the optic part 310, but not limited thereto. It is only necessary to provide the support parts in a pair and make each support part foldable halfway.

### [Reference signs]

- 1: Intraocular lens injection instrument (injector)
- 10: Cartridge
- 20: Cylindrical unit
- 30: Push-out member (Plunger)
- 100, 200: Intraocular lens
- 110, 210: Optic part
- 120, 220: Support part
- 121: Root
- 122: Arm
- 123: Constricted portion
- 212a: Recessed portion

## Claims

1. A foldable intraocular lens (300) made of soft resin and comprising an optic part (310) and a pair of support parts (320) integrally formed with the optic part (310) for supporting the optic part in an eye, each support part (320) including a root (R) formed to extend from a periphery of the optic part (310) and located at positions to each other with respect to a center of the optic part (310), each root (R) radially extending by a predetermined length in a first direction; and an arm (321a, 321b) extending in a curve from each root (R) in a second direction, wherein
each arm (321a, 321b) includes a rear end portion (321b) joined to each root (R) and a distal end portion (321a) joined to each rear end portion (321b), each distal end portion (321a) having a distal end (P) of each arm;
a first constricted portion (326) is formed between each root (R) and each rear end portion (321b);
a second constricted portion (325) that is formed between each first constricted portion (326) and each distal end (P), each constricted portion (325, 326) formed on a side of each arm (321a, 321b) facing the optic part (310) to inwardly fold the arm by a predetermined length from a distal end (P) thereof, and
each support part (320) is foldable at each constricted portion (325, 326) serving as a base point when each arm (321a, 321b) is subjected to stress from a push rod (31) of an injector (1) during a push-out operation of the intraocular lens by use of the injector (1).

2. The intraocular lens according to claim 1, wherein
the first direction is a radial direction from the optical axis of the optic part (310).

3. The intraocular lens according to claim 1 or 2, wherein
a line segment joining the center of the optic part (310) to a midpoint of each root (R) forms an angle of 50° or more and 90° or less with a line segment joining the center of the optic part (310) to the distal end (P) of each arm.

4. The intraocular lens according to one of claims 1 to 3, wherein
each arm is located on a more front surface side of the optic part (310) than the peripheral edge of the optic part (310).

5. The intraocular lens according to one of claims 1-4, wherein
a width or thickness of each distal end portion (321a) is smaller than a width or thickness of each rear end portion (321b).

6. The intraocular lens according to one of claims 1-5, wherein a width or a cross sectional area of each second constricted portion (325) is smaller than a width or a cross sectional area of each first constricted portion (326).

## Patentansprüche

1. Faltbare Intraokularlinse (300) aus Weichharz und aufweisend ein optisches Teil (310) und ein Paar von Stützteilen (320), die integral mit dem optischen Teil (310) ausgebildet sind, zum Stützen des optischen Teils in einem Auge, wobei jedes Stützteil (320) eine Wurzel (R), die so ausgebildet ist, dass sie sich von einem Umfang des optischen Teils (310) erstreckt, und die an bezüglich einer Mitte des optischen Teils (310) einander gegenüber liegenden Positionen ange-ordnet sind, wobei jede Wurzel (R) eine vorbestimmte Länge in einer ersten Richtung radial verläuft, und einen Arm (321a, 321b), der sich in einer Kurve von der jeweiligen Wurzel (R) in einer zweiten Richtung erstreckt, enthält, wobei
jeder Arm (321a, 321b) einen hinteren Endabschnitt (321b), der mit der jeweiligen Wurzel (R) verbunden ist, und einen distalen Endabschnitt (321a), der mit dem jeweiligen hinteren Endabschnitt (321b) verbunden ist, enthält, wobei jeder distale Endabschnitt (321a) ein distales Ende (P) des jeweiligen Arms aufweist;
ein erster eingeschnürter Abschnitt (326) zwischen der jeweiligen Wurzel (R) und dem jeweiligen hinteren Endabschnitt (321b) ausgebildet ist;
ein zweiter eingeschnürter Abschnitt (325) zwischen dem jeweiligen ersten eingeschnürten Abschnitt (326) und dem jeweiligen distalen Ende (P) ausgebildet ist, wobei jeder eingeschnürte Abschnitt (325, 326) auf einer Seite des jeweiligen Arms (321a, 321b) ausgebildet ist, die dem optischen Teil (310) zugewandt ist, um den Arm eine vorbestimmte Länge von einem distalen Ende (P) davon nach innen zu falten; und
jedes Stützteil (320) an dem jeweiligen eingeschnürten Abschnitt (325, 326) als Basispunkt faltbar ist, wenn auf den jeweiligen Arm (321a, 321b) von einer Schubstange (31) eines Injektors (1) während eines Ausdrückvorgangs der Intraokularlinse mittels des Injektors (1) Druck ausgeübt wird.

2. Intraokularlinse nach Anspruch 1, bei welcher die erste Richtung eine radiale Richtung von der optischen Achse des optischen Teils (310) ist.

3. Intraokularlinse nach Anspruch 1 oder 2, bei welcher ein Liniensegment, das die Mitte des optischen Teils (310) mit einem Mittelpunkt jeder Wurzel (R) verbindet, einen Winkel von 50° oder mehr und 90° oder weniger mit einem Liniensegment, das die Mitte des optischen Teils (310) mit dem distalen Ende (P) jedes Arms verbindet, bildet.

4. Intraokularlinse nach einem der Ansprüche 1 bis 3, bei welcher
jeder Arm weiter zur vorderen Oberflächenseite des optischen Teils (310) positioniert ist als die Umfangskante des optischen Teils (310).

5. Intraokularlinse nach einem der Ansprüche 1 bis 4, bei welcher
eine Breite oder Dicke jedes distalen Endabschnitts (321a) kleiner als eine Breite oder Dicke jedes hinteren Endabschnitts (321b) ist.

6. Intraokularlinse nach einem der Ansprüche 1 bis 5, bei welcher
eine Breite oder eine Querschnittsfläche jedes zweiten eingeschnürten Abschnitts (325) kleiner als eine Breite oder eine Querschnittsfläche jedes ersten eingeschnürten Abschnitts (326) ist.

## Revendications

1. Lentille intraoculaire pliable (300) réalisée en une résine souple et comprenant une partie optique (310) et une paire de parties de support (320) formées de manière unitaire avec la partie optique (310) afin de supporter la partie optique sur un oeil, chaque partie de support (320) comportant une racine (R) formée de manière à s'étendre depuis une périphérie de la partie optique (310), et situées à des emplacements opposés l'un à l'autre par rapport à un centre de la partie optique (310), chaque racine (R) s'étendant radialement sur une longueur prédéterminée dans une première direction ; et un bras (321a, 321b) s'étendant suivant une courbe à partir de chaque racine (R) dans une seconde direction, dans laquelle
chaque bras (321a, 321b) comporte une partie d'extrémité arrière (321b) reliée à chaque racine (R) et une partie d'extrémité distale (321a) reliée à chaque partie d'extrémité arrière (321b), chaque partie d'extrémité distale (321a) comportant une extrémité distale (P) de chaque bras ;
une première partie restreinte (326) est formée entre chaque racine (R) et chaque partie d'extrémité arrière (321b) ;
une seconde partie restreinte (325) qui est formée entre chaque première partie restreinte (326) et chaque extrémité distale (P), chaque partie restreinte (325, 326) étant formée sur un côté de chaque bras (321a, 321b) faisant face à la partie optique (310) afin de plier vers l'intérieur le bras sur une longueur prédéterminée à partir d'une extrémité distale (P) de celui-ci, et
chaque partie de support (320) peut être repliée au niveau de chaque partie restreinte (325, 326) servant de point de base lorsque chaque bras (321a, 321b) est soumis à une contrainte à partir d'une tige de poussée (31) d'un injecteur (1) au cours d'une opération d'éjection de la lentille intraoculaire en utilisant l'injecteur (1).

2. Lentille intraoculaire selon la revendication 1, dans laquelle
la première direction est une direction radiale à partir de l'axe optique de la partie optique (310).

3. Lentille intraoculaire selon la revendication 1 ou 2, dans laquelle
un segment de ligne reliant le centre de la partie optique (310) à un point central de chaque racine (R) forme un angle supérieur ou égal à 50° et inférieur ou égal à 90° par rapport à un segment de ligne reliant le centre de la partie optique (310) à l'extrémité distale (P) de chaque bras.

4. Lentille intraoculaire selon l'une des revendications 1 à 3, dans laquelle
chaque bras est situé sur un côté de surface plus en avant de la partie optique (310) que le bord périphérique de la partie optique (310).

5. Lentille intraoculaire selon l'une des revendications 1 à 4, dans laquelle
une largeur ou épaisseur de chaque partie d'extrémité distale (321a) est inférieure à une largeur ou épaisseur de chaque partie d'extrémité arrière (321b).

6. Lentille intraoculaire selon l'une des revendications 1 à 5, dans laquelle une largeur ou une surface de section transversale de chaque seconde partie restreinte (325) est inférieure à une largeur ou une surface de section transversale de chaque première partie restreinte (326).
